# EUROPEAN PATENT APPLICATION

(11) **EP 4 806 343 A1**
(43) Date of publication of application: **16.09.2026**
(21) Application number: 26162993.5
(22) Date of filing: 06.03.2026
(51) Int. Cl.: A61B 5/021, A61B 5/024, A61B 5/0535, A61B 5/00

(54) **COMPUTING DEVICE HAVING MINIATURIZED IMPEDANCE PLETHYSMOGRAPHY SENSOR FOR MEASURING BLOOD FLOW**

(30) Priority: 14.03.2025 US 202519080266
(71) Applicant: Google LLC, Mountain View, CA 94043 (US)
(72) Inventor: Thomson, Seamus David, Mountain View, 94043 (US); Jung, Seobin, Mountain View, 94043 (US); Mukherjee, Debanjan, Mountain View, 94043 (US); Narasimhan, Ravi, Mountain View, 94043 (US); Patel, Shwetak, Mountain View, 94043 (US); Sheng, Samuel Wei, Mountain View, 94043 (US)
(74) Representative: Maikowski & Ninnemann Patentanwälte Partnerschaft mbB

(57) **Abstract**

A user computing device includes a housing and an impedance plethysmography (IPG) sensor positioned on the housing. The IPG sensor has a pair of miniaturized excitation electrodes and a pair of miniaturized sensing electrodes that together are sized to fit under a fingertip of a user. Further, the IPG sensor is configured to generate IPG data indicative of an impedance at the fingertip of the user with respect to time. The user computing device also includes a processor configured to receive the IPG data, generate a pulsatile signal for deriving a blood pressure or a proxy of blood pressure of the user based at least in part on the IPG data, and determine the blood pressure or the proxy of blood pressure of the user based on the pulsatile signal.

## Description

### FIELD OF THE INVENTION

The present disclosure relates generally to computing devices for measuring blood flow, and more particularly, to computing devices having one or more miniaturized impedance plethysmography (IPG) sensors for measuring blood flow, e.g., at the fingertip.

### BACKGROUND

Measuring blood flow on the fingertip is an exciting area of research, especially within the domain of blood pressure, because optical-based sensing methods - such as photoplethysmography (PPG) - on the fingertip have yielded promising results for blood pressure estimation or hypertension screening. Despite this prospect, however, fingertip PPG sensing encounters several challenges from both a physiological sensing and applicability to consumer products perspective.

Physiologically, PPG signals typically represent blood flow at the superficial tissue level, i.e., measuring blood flow in capillaries near the surface of the skin. This renders the signal sensitive to various confounding factors known to impact both the signal quality and the waveform morphology. Some of these confounders include vasoconstriction and dilation, as well as applied pressure to the skin. If one tries to rely on fingertip PPG signals as it is to read hemodynamic information, signal sensitivity to these confounding factors are a key aggressor towards deriving accurate blood pressure readings.

While oscillometric methods (which apply pressure to the fingertip and analyze the pressure-modulated PPG signal) offer an alternative, these methods introduce a different challenge. For instance, it can become difficult to distinguish between arterial and capillary collapse under the applied pressure, impacting the reliability of blood pressure estimations. In addition, from a consumer product perspective, introducing a PPG sensing module into a small region on a hardware product suitable for fingertip placement and sensing is challenging: optical architecture requires multiple components and material layers (e.g. optically transparent materials, optical filters, lens, photodiodes, light emitting diodes, electrical architecture, etc.), and finding appropriate real estate for this sensor for a natural user experience (e.g. on a button) is limited.

Overall, there are recognized challenges with deriving a fingertip PPG sensor suitable for accurate blood pressure measurement on a consumer product. As such, the present disclosure is directed to a computing device that addresses these aforementioned challenges by defining a bioimpedance sensing approach for measuring a pulsatile waveform on the fingertip within a confined space suitable for consumer products.

### SUMMARY OF THE INVENTION

Aspects and advantages of embodiments of the present disclosure will be set forth in part in the following description, or can be learned from the description, or can be learned through practice of the embodiments.

In an aspect, the present disclosure is directed to a user computing device that includes a housing and an impedance plethysmography (IPG) sensor positioned on the housing. The IPG sensor has a pair of miniaturized excitation electrodes and a pair of miniaturized sensing electrodes that together are sized to fit under a fingertip of a user. Further, the IPG sensor is configured to generate IPG data indicative of an impedance at the fingertip of the user with respect to time. The user computing device also includes a processor configured to receive the IPG data, generate a pulsatile signal for deriving a blood pressure or a proxy of blood pressure of the user based at least in part on the IPG data, and determine the blood pressure or the proxy of blood pressure of the user based on the pulsatile signal.

In another aspect, the present disclosure is directed to a method for monitoring blood pressure of a user. The method includes generating, via an impedance plethysmography (IPG) sensor of a user computing device, IPG data indicative of an impedance at a fingertip of a user with respect to time, the IPG sensor having a pair of miniaturized excitation electrodes and a pair of miniaturized sensing electrodes that together are sized to fit under the fingertip of the user, generating, via a processor, a pulsatile signal for deriving the blood pressure of the user based at least in part on the IPG data, and determining, via the processor, the blood pressure of the user based on the pulsatile signal.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 illustrates a front view of a user computing device according to an embodiment of the present disclosure;
FIGS. 2A-2C illustrate schematic views of a user computing device having an impedance plethysmography (IPG) sensor according to an embodiment of the present disclosure;
FIGS. 3A-3C illustrate schematic views of a user computing device having an IPG sensor according to another embodiment of the present disclosure;
FIGS. 4A-4C illustrate schematic views of a user computing device having an IPG sensor according to still another embodiment of the present disclosure;
FIGS. 5A and 5B illustrate schematic views of a user computing device having an IPG sensor according to yet another embodiment of the present disclosure;
FIGS. 6A-6D illustrate various views of different user computing devices having an IPG sensor according to embodiments of the present disclosure;
FIG. 7 illustrates various components of a computing system that can be utilized according to an embodiment of the present disclosure;
FIG. 8 illustrates a schematic diagram of an example set of devices that are able to communicate according to an embodiment of the present disclosure; and
FIG. 9 illustrates a flow diagram of an example, non-limiting computer-implemented method for monitoring blood pressure of a user according to example embodiments of the present disclosure.

Reference numerals that are repeated across plural figures are intended to identify the same features in various implementations.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope or spirit of the invention. For instance, features illustrated or described as part of an embodiment can be used with another embodiment to yield a still further embodiment. Thus, it is intended that the present invention covers such modifications and variations as come within the scope of the appended claims and their equivalents.

Generally, the present disclosure is directed to using a miniaturized IPG sensor of a user computing device to monitor blood pressure. For instance, the present disclosure is directed to a miniaturized, localized IPG sensor limited to a small sensing area. Thus, in an embodiment, the IPG sensor may be a dry-electrode-based bioimpedance sensor for acquiring a pulsatile signal at a user's fingertip suitable for consumer products. As such, the IPG sensor is designed so that the sensor can operate in confined consumer product real estate appropriate for seamless user interactions - such as that of a smartphone side button. In particular embodiments, the IPG sensor of the present disclosure includes four electrodes - two outer electrodes for injecting a small alternating current, and two inner electrodes for measuring the resulting voltage. By measuring the impedance on a fingertip with respect to time, a pulsatile waveform can be generated and used to derive features relevant for blood pressure estimation. Further, in an embodiment, the IPG sensor may be primarily constructed of two materials, such as a conductive material (e.g., the electrodes) and an insulating substrate material. Thus, in such embodiments, the insulating material is configured to prevent electrical shorting between the electrodes, as well as with the casing/housing or internal electronics. In some embodiments, the electrodes may be slightly raised (e.g., on the order of from about 10s to about 100s of microns) relative to the insulating substrate, so that fingertip-to-electrode contact is facilitated.

The user computing device may further include electrical circuitry accessing the electrodes. In an embodiment, for example, the electrical circuitry may pass through the insulating substrate to make contact with the electrodes, e.g., on the non-fingertip side. In such embodiments, the electrical circuitry is configured to connect to an electrical architecture capable of performing bioimpedance/IPG calculations, such as that of an analogue front end (AFE) device.

In some embodiments, the user computing device may also include an optical module capable of photoplethysmography (PPG) sensing. More particularly, in an embodiment, the optical module may be positioned centrally, e.g., between the sensing IPG electrodes such that simultaneous measurements (e.g., both optical and impedance based) are collocated and the waveform features for each respective sensor are representative of a similar tissue region. In another embodiment, the optical module capable of PPG sensing may be positioned underneath the IPG electrodes. In an embodiment, in order for a PPG signal to be acquired with this module, the electrodes may need to have conductive and transparent material properties - such as those of transparent conducting films (TCFs). Further, in certain embodiments, the electrical connections to the transparent electrodes may be unable to directly pass through the optical module; and would thus be connected to the electrodes from a different location, such as an electrode underside edge. In such embodiments, this different location allows for sufficient clearance for the electrical connection to occur wherein it does not directly interfere with the optical path.

In additional embodiments, the IPG sensor may also be utilized on further devices (in addition to a smartphone), such as a side button surface of a wearable computing device, an externally facing surface of earbuds, and/or any other locations on a smartphone independent to that of side buttons. Furthermore, in an embodiment, the miniaturized IPG sensor(s) of the present disclosure may also be relevant for headsets, such as those used in augmented reality (AR) and/or virtual reality (VR) head-positioned hardware and/or controllers. For the head-positioned hardware, in an embodiment, as these devices are typically positioned over the head (e.g., covering hair), there are few areas for direct skin-contact sensing, predominantly on the face (e.g., at the temples). Accordingly, the miniaturized IPG sensor design described herein may also be suitable for positioning in these head areas for IPG signal acquisition during AR/VR experiences.

It should be further understood that though the IPG sensor described herein can be used for monitoring blood flow at the fingertip, such sensors may also be utilized in other areas having suitable blood flow properties, e.g., areas in which deeper blood flow is detectable in arteries rather than blood flow in capillaries alone. Such areas, for example, may include the neck (carotid arteries), wrist (radial artery), upper arm (brachial artery), groin (femoral artery), behind the knee (popliteal artery), on the top of the foot (dorsalis pedis artery), and the temples (superficial temporal artery), as these locations have arteries close to the skin surface that can be palpated to feel a pulse.

With reference now to the figures, example embodiments of the present disclosure will be discussed in further detail.

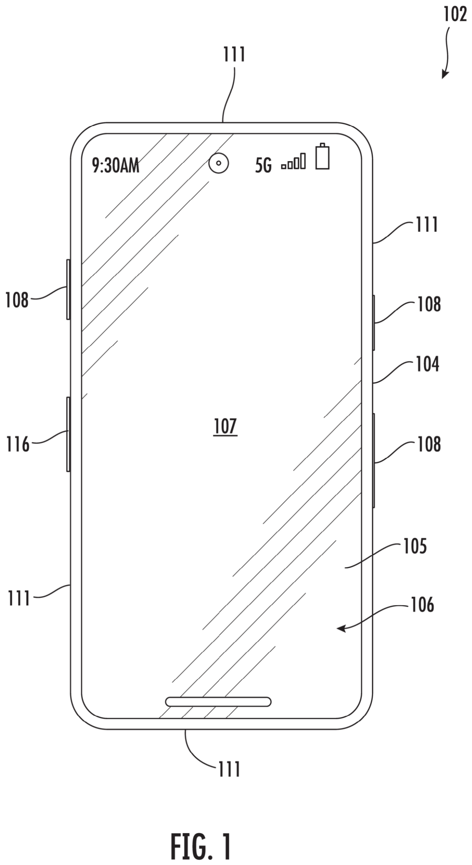

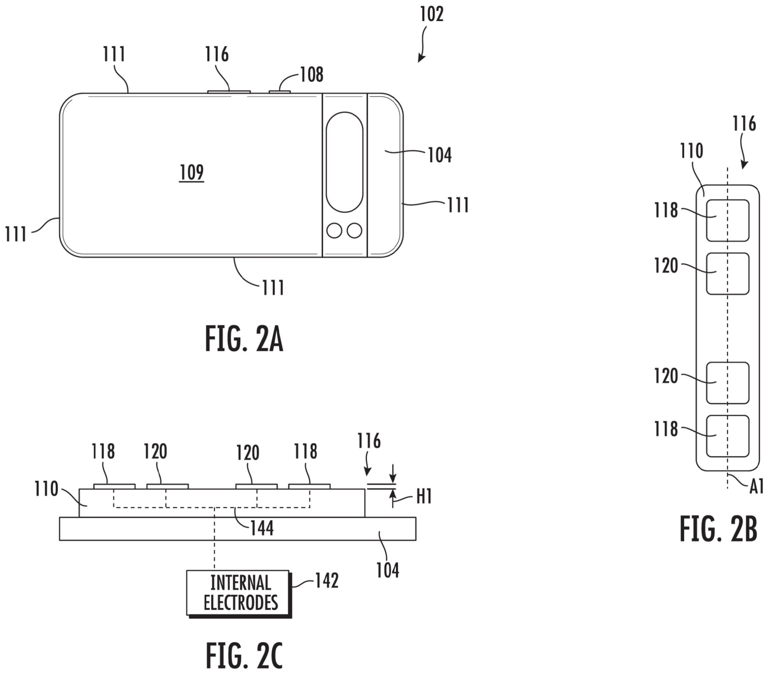

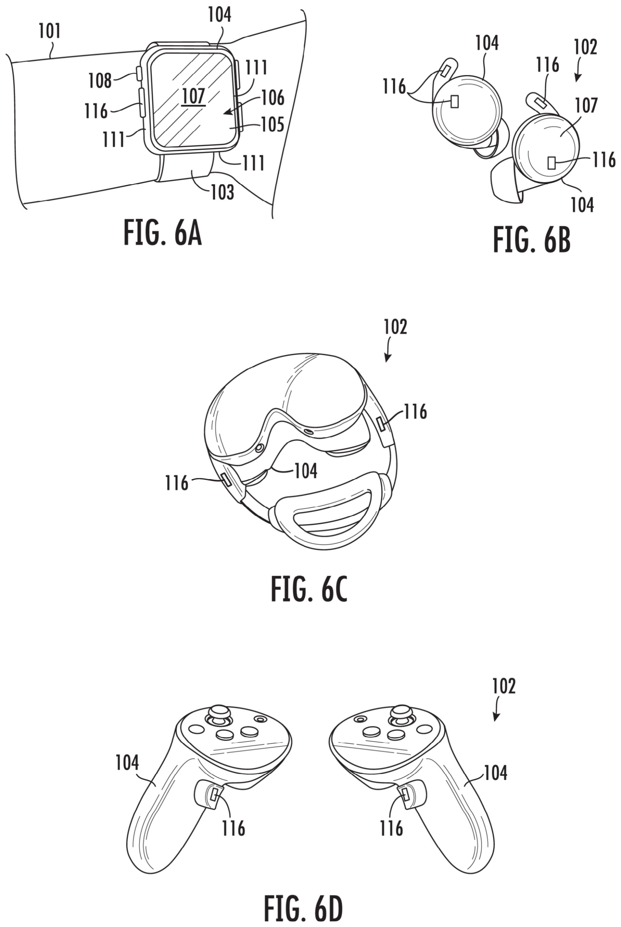

Referring now to the drawings, FIGS. 1-6D illustrate various views of example user computing devices 102 according to the present disclosure. In particular, as shown, the user computing device 102 generally includes a housing 104 that defines an interior volume for containing electronics associated with the user computing device 102. Furthermore, the housing 104 may generally define an upper side 107 (FIGS. 1, 6A and 6B), a lower side 109 (FIGS. 2A, 3A, and 4A), and a plurality of side walls 111 (FIGS. 1, 2A, 3A, 4A, 5A, and 6A). Moreover, the user computing device 102 has an outer covering 105 on an upper side for enclosing the interior volume. In an embodiment, the outer covering 105 may be constructed of glass, polycarbonate, acrylic, or similar. Further, as shown in FIGS. 1 and 6A, the user computing device 102 includes an electronic display screen 106 arranged within the housing 104 and viewable through the outer covering 105. The electronic display screen 106 may cover an electronics package (not shown), which may also be housed within the housing 104. The display 106 may be any suitable display, such as a touch screen, organic light emitting diode (OLED), or liquid crystal display (LCD). Moreover, as generally shown in FIGS. 1-6A, the user computing device 102 may also include one or more buttons 108 that may be implemented to provide a mechanism to activate various mechanisms and/or sensors of the user computing device 102, such as to collect certain health data of the user. Thus, it should be generally understood that the user computing device 102 may be a smartphone (as shown in FIGS. 1-5A), a wearable computing device, such as a smartwatch (as shown in FIG. 6A), a tablet computer, a laptop, earbuds (FIG. 6B), a headset (FIG. 6C), headphones, gaming controllers (FIG. 6D), and/or any other suitable user computing device.

Thus, in particular embodiments, as shown in FIG. 6A, wherein the user computing device 102 is a smartwatch, the user computing device 102 may be worn on a user or wearer's forearm 101 like a wristwatch. In such embodiments, the user computing device 102 may include a wristband 103 for securing the user computing device 102 to the user or wearer's forearm 101. However, it should be appreciated that the user computing device 102 may be worn at any other suitable location by a user, such as, for example, on an ankle. It should be appreciated that the user computing device 102 can include a ring, band, earring, necklace, or any other suitable wearable computing device known by one of skill in the art.

Referring generally to FIGS. 1-6D, the user computing device 102 further includes one or more miniaturized impedance plethysmography (IPG) sensors 116 positioned on the housing 104 for generating IPG data. For example, in an embodiment, the IPG sensor(s) 116 may be positioned on the upper side 107, one of the plurality of side walls 111, and/or the lower side 109 of the user computing device 102. Furthermore, as shown particularly in FIGS. 2A-5B, each IPG sensor 116 may include at least four electrodes constructed of a conductive material and mounted an insulating substrate material 110. Thus, as shown, the insulating substrate material 110 of the IPG sensor(s) 116 may be positioned on the housing 104 (e.g., such that the electrodes are easily accessible to a user).

In particular embodiments, as shown, the four electrodes may include a pair of miniaturized excitation electrodes 118 and a pair of miniaturized sensing electrodes 120 that together are sized to fit under a fingertip of a user. Furthermore, as shown, the insulating substrate material 110 may be arranged between the electrodes 118, 120 and the user computing device 102. In such embodiments, the insulating substrate material 110 is configured to mitigate electrical shorting between the miniaturized excitation electrodes 118, the miniaturized sensing electrodes 120, the housing 104, and/or one or more internal electronics of the user computing device 102.

Referring particularly to FIGS. 2C, 3C, and 4C, the miniaturized excitation electrodes 118 and the miniaturized sensing electrodes 120 may be raised with respect to the insulating substrate material 110 by a certain height H1. In such embodiments, for example, the certain height may range from 10s of microns to 100s microns.

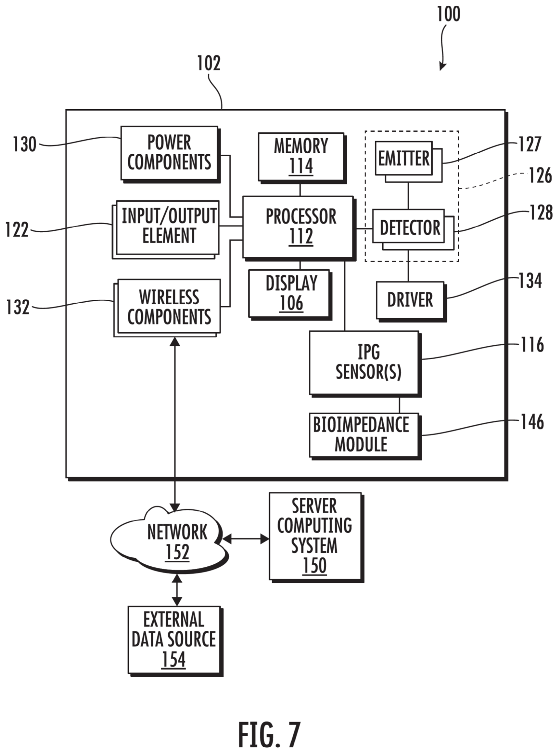

Moreover, in an embodiment, the IPG sensor(s) 116 described herein are configured to generate IPG data indicative of an impedance at the fingertip of the user with respect to time. More specifically, in an embodiment, the miniaturized excitation electrodes 118 are configured to inject alternating current, whereas the miniaturized sensing electrodes 120 are configured to measure the resulting voltage potential due to the current applied across the excitation electrodes 118. Accordingly, the user computing device 102 described herein may also include a processor 112 (FIG. 7) configured to receive the IPG data, generate a pulsatile signal for deriving a blood pressure or a proxy of blood pressure of the user based at least in part on the IPG data, and determine the blood pressure or the proxy of blood pressure of the user based on the pulsatile signal. Thus, in such embodiments, the blood pressure or proxies thereof may include but are not limited to absolute blood pressure, hypertension screening, blood pressure trends over time, blood pressure variability, and/or nocturnal blood pressure dip (e.g., decreases in blood pressure during sleep), etc.

In further embodiments, the pair of miniaturized excitation electrodes 118 may be arranged on the exterior of the pair of miniaturized sensing electrodes 120. For example, in some instances, as shown in FIGS. 2B, 2C, 3B, 3C, 4B, 4C, and 5B, the miniaturized sensing electrodes 120 may be positioned between the miniaturized excitation electrodes 118 such that miniaturized excitation electrodes 118 are spaced apart by the miniaturized sensing electrodes 120. In one or more instances, the IPG sensor 116 may include more than two pairs of sensing electrodes 120 for generating IPG data. In such instances, the two pairs of sensing electrodes 120 used to generate IPG data may be selected based at least in part on signal quality of the IPG data.

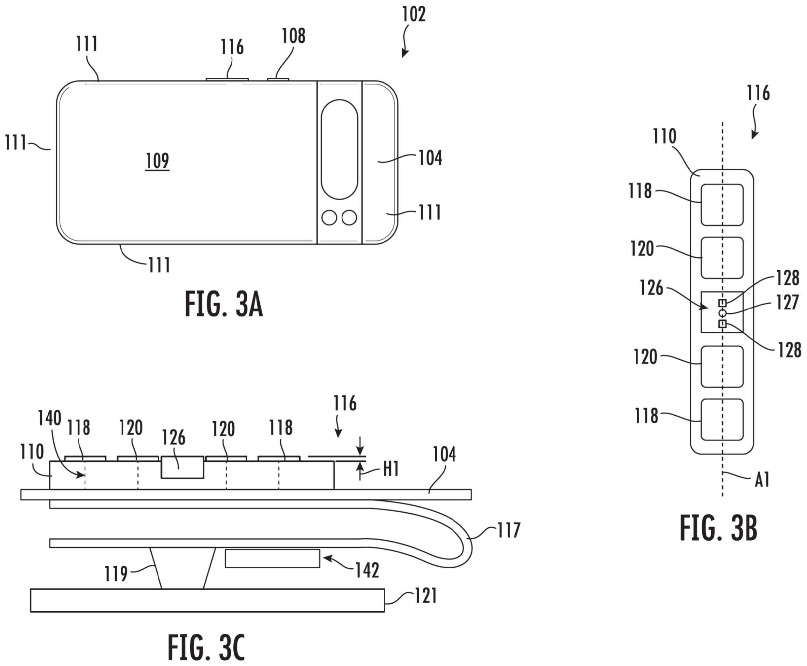

In additional embodiments, as shown in FIGS. 2C and 3C, the user computing device 102 further includes electrical circuity 140 electrically coupled with the electrodes 118, 120 that passes through the insulating substrate material 110 to internal electrodes 142 (e.g., an analog front end chip for IPG, PPG, or both) on a non-fingertip side of the IPG sensor 116. Moreover, as shown, the user computing device 102 may include a flexible printed circuit board 117, a button module 119, and a rigid printed circuit board 121, e.g., within the housing 104. Thus, in such embodiments, the electrical circuitry 140 may be further electrically coupled with a bioimpedance module 146 (FIG. 7) performing bioimpedance (e.g., IPG calculations).

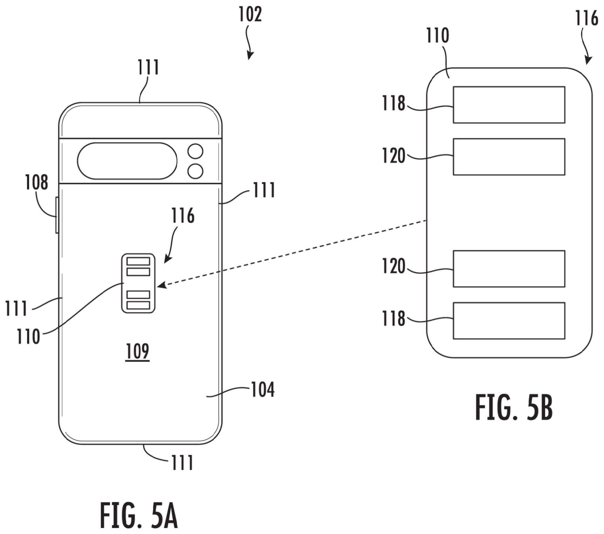

In addition, as shown in FIGS. 2B, 2C, 3B, 3C, 4B, 4C, and 5B, the miniaturized excitation electrodes 118 and the miniaturized sensing electrodes 120 may be arranged in a linear pattern, e.g., may be aligned on a common axis A1. However, in other instances, the miniaturized excitation electrodes 118 and the miniaturized sensing electrodes 120 may be positioned in any other suitable pattern. Furthermore, in an embodiment, the IPG sensor(s) 116 may be positioned on the upper side 107 of the user computing device 102, one of the plurality of side walls 111, and/or the lower side 109. For example, as shown in FIGS. 1-4C and 6A, the IPG sensor(s) 116 is positioned on one of the side walls 111 of the user computing device 102. In FIGS. 5A and 5B, the IPG sensor(s) 116 is positioned on the lower side 109 of the user computing device 102. In other embodiments, as shown in FIG. 6B, the IPG sensor(s) 116 may be positioned at any suitable location on earbuds (e.g., wherein a user can touch the earbuds while being worn or while holding). Moreover, as shown in FIGS. 6C and 6D, the IPG sensor(s) 116 may be positioned at any suitable location on augmented reality (AR) and/or virtual reality (VR) head-positioned hardware and/or controllers. It should be generally understood by those having ordinary skill in the art that the sensors described herein may generally be positioned at any other suitable location on the user computing device 102 such that the sensors are able to be selectively (e.g., "actively") contacted by a user for taking an active measurement, e.g. of blood pressure, and/or may also be configured to collect data passively (e.g., without requiring engagement by the user).

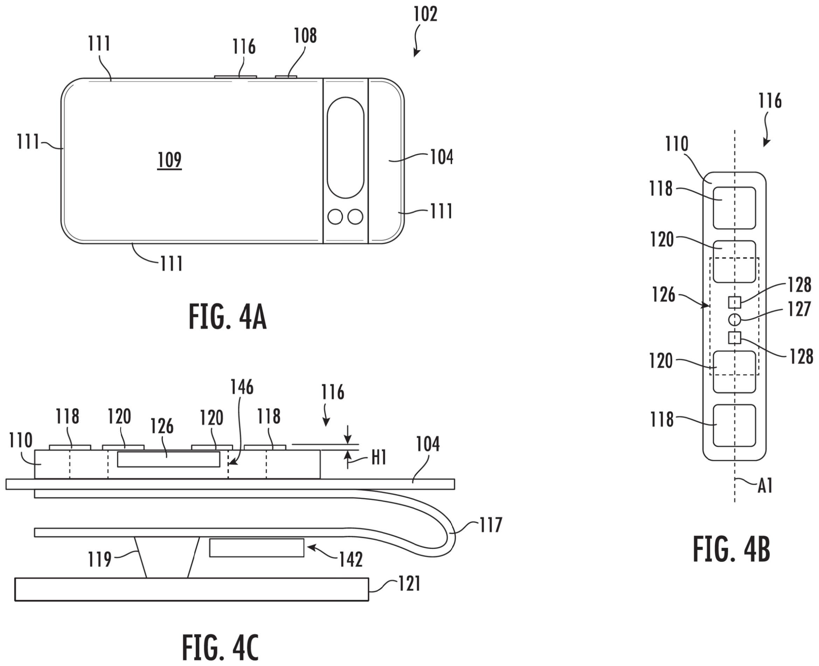

Further, as generally shown in FIGS. 3B, 3C, 4B, and 4C, the user computing device 102 may include an optical module having one or more photoplethysmography (PPG) sensors 126 on the housing 104. As shown in FIGS. 3B and 4B, the PPG sensor(s) 126 has one or more emitters 127, such as one or more light-emitting diodes (LEDs), for emitting controlled pulses of light and has one or more detectors 128, such as photodiodes, to generate data indicative of the detected, returned light. Some PPG technologies rely on detecting light at a single spatial location, adding signals taken from two or more spatial locations, or an algorithmic combination thereof. Both of these approaches result in a single spatial measurement from which physiological metrics can be determined. In some embodiments, the PPG sensor(s) 126 employs a single emitter 127 associated with a single detector 128 (i.e., a single light path). Additionally, or alternatively, the PPG sensor(s) 126 may employ multiple emitters 127 coupled to a single detector 128 or multiple detectors 128 (i.e., two or more light paths). In other embodiments, the PPG sensor(s) 126 may additionally, or alternatively, employ multiple detectors 128 coupled to a single light emitter 127 or multiple emitters 127 (i.e., two or more light paths).

Accordingly, in an embodiment, the processor 112 is configured to control the emitter(s) 127 to emit photons, which reflect off the skin, tissue, bones, blood, etc. of a user for detection by the detectors 128, and converts the analog current received from the detector(s) 128 into a digital PPG signal. The PPG sensor(s) 126 can be configured for use at various light wavelengths, such as green (centered at 528 nanometers (nm)), red (centered at 660 nm), and infrared (centered at 940 nm), where the amplitude of the reflected light for the green, red, and infrared wavelengths changes with every heartbeat. Typically, the peak to peak amplitude for green PPG signals is greater than the peak to peak amplitude for red PPG signals and infrared PPG signals when there is a clear pulsatile signal such as that associated with a heartbeat. In some cases, a PPG sensor 126 may employ a single light emitter 127 and two or more light detectors 128 each configured to detect a specific wavelength or wavelength range. In some cases, each detector 128 is configured to detect a different wavelength or wavelength range from the other detectors 128. In other cases, two or more detectors 128 are configured to detect the same wavelength or wavelength range. In yet another case, one or more detectors 128 is configured to detect a specific wavelength or wavelength range different from one or more other detectors 128). In embodiments employing multiple light paths, the PPG sensor(s) 126 may determine an average of the signals resulting from the multiple light paths before determining an HR estimate or other physiological metrics. In any event, the PPG sensor(s) 126 is usable to generate non-invasive biometric data.

In particular embodiments, as shown in FIGS. 3B, 3C, 4B, and 4C, the PPG sensor(s) 126 may be disposed proximate the IPG sensor(s) 116 to ensure both the PPG sensor(s) 126 and the IPG sensor(s) 116 are generating data indicative for the same anatomical region. For instance, in an embodiment, as shown in FIGS. 3A and 3B, the PPG sensor(s) 126 is positioned centrally within the IPG sensor 116, e.g., between the pair of miniaturized excitation electrodes 118 and the pair of miniaturized sensing electrodes 120. In other embodiments, as shown in FIGS. 4B and 4C, the PPG sensor 126 may be positioned below at least a portion of the IPG sensor(s) 116. More specifically, as shown, the PPG sensor 126 extends at least partially below the pair of miniaturized sensing electrodes 120.

In an embodiment, in order for a PPG signal to be acquired with this module, the miniaturized excitation electrodes 118 and/or the miniaturized sensing electrodes 120 are required to be electrically conductive (so that they can serve as electrodes), and have an optical transmissivity high enough such that it does not block light paths from and/or to the PPG module, such as those of transparent conducting films (TCFs). In this example, electrical circuitry 144 electrically coupling the transparent electrodes 118, 120 to the internal electrodes 142 is unable to directly pass through the optical module having the PPG sensor 126. Thus, as shown in FIG. 4C, the electrical circuitry 144 may be connected to certain of the transparent electrodes 120 (e.g., the excitation electrodes 120) from a different location, e.g., an electrode side edge 146 to provide sufficient clearance for the electrical circuitry 144 to occur without directly interfering with the optical path.

Referring now to FIG. 7, components of an example computing system 100 of the user computing device 102 that can be utilized in accordance with various embodiments are illustrated. In particular, as shown, the computing system 100 may also include at least one processor 112 communicatively coupled to different parts of the user computing device 102, such as the display 106, the IPG sensor(s) 116, the PPG sensor(s) 126, and any other sensors present. Moreover, in an embodiment, the processor(s) 112 may be a central processing unit (CPU) or graphics processing unit (GPU) for executing instructions that can be stored in a memory 114, such as flash memory or DRAM, among other such options. For example, in an embodiment, the memory 114 may include RAM, ROM, FLASH memory, or other non-transitory digital data storage, and may include a control program comprising sequences of instructions which, when loaded from the memory 114 and executed using the processor(s) 112, cause the processor(s) 112 to perform the functions that are described herein. As would be apparent to one of ordinary skill in the art, the computing system 100 can include many types of memory, data storage, or computer-readable media, such as data storage for program instructions for execution by any suitable processor. The same or separate storage can be used for images or data, a removable memory can be available for sharing information with other devices, and any number of communication approaches can be available for sharing with other devices.

The computing system 100 also includes one or more power components 130, such as a battery operable to be recharged through conventional plug-in approaches, or through other approaches such as capacitive charging through proximity with a power mat or other such device. Moreover, as shown, the computing system 100 may also include one or more wireless components 132 operable to communicate with one or more electronic devices within a communication range of the particular wireless channel. The wireless channel can be any appropriate channel used to enable devices to communicate wirelessly, such as Bluetooth, cellular, NFC, Ultra-Wideband (UWB), or Wi-Fi channels. It should be understood that the computing system 100 can have one or more conventional wired communications connections as known in the art.

The computing system 100 may be configured to receive inputs from the display 106 (e.g., when the display 106 is a touch screen) and/or to control the display 106 to convey information, although devices might convey information via other means, such as through audio speakers, projectors, or casting the display or streaming data to another device, such as a mobile phone, wherein an application on the mobile phone displays the data. In further embodiments, the computing system 100 can also include at least one additional input-output (I/O) component 122 able to receive conventional input from a user. This conventional input can include, for example, a push button, touch pad, touch screen, wheel, joystick, keyboard, mouse, keypad, or any other such device or element whereby a user can input a command to the computing system 100.

In another embodiment, the I/O component(s) 122 may be connected by a wireless infrared or Bluetooth or other link as well in some embodiments. In some embodiments, the computing system 100 may also include a microphone or other audio capture element that accepts voice or other audio commands. For example, in particular embodiments, the computing system 100 may not include any buttons at all but might be controlled only through a combination of visual and audio commands, such that a user can control the user computing device 102 without having to be in contact therewith. In certain embodiments, the I/O components 122 may also include one or more of the IPG sensor(s) 116 described herein, optical sensors (e.g., PPG sensor(s) 126), and the like.

It should be appreciated that emitters 127 and detectors 128 of the PPG sensor(s) 126 may be coupled to the processor 112 directly or indirectly using driver circuitry 134 by which the processor 112 may control the emitters 127 to emit light and obtain signals from the detectors 128. Similarly, the excitation electrodes 118 of the IPG sensor(s) 116 may be coupled to the processor 112 directly or indirectly for supplying current (e.g., from the power component(s) 130) across the excitation electrodes 118.

Further, as shown, the computing system 100 may also include a server computing system 150 can communicate with wireless components 132 via one or more networks 152, which may include one or more local area networks, wide area networks, UWB, and/or internetworks using any of terrestrial or satellite links. In some embodiments, the server computing system 150 executes control programs and/or application programs that are configured to perform some of the functions described herein. Moreover, the network(s) 152 may allow one or more other devices(s) to communicate with the user computing device 102, such as one or more external data source(s) 154 (e.g., for providing blood pressure data, and/or the like).

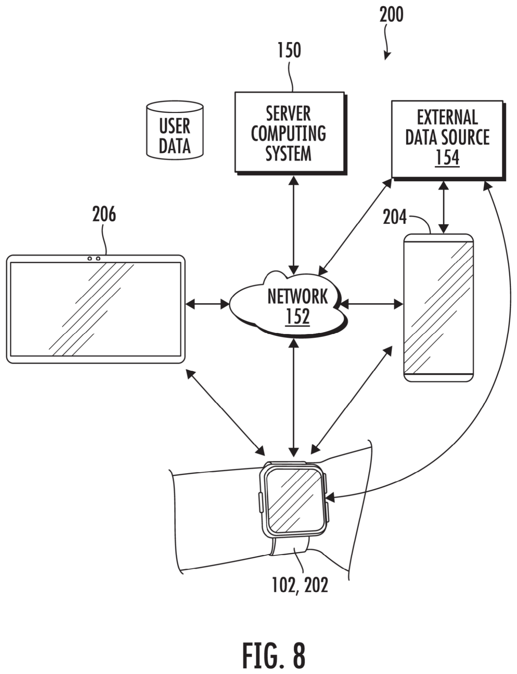

For instance, referring now to FIG. 8, a schematic diagram of an environment 200 in which aspects of various embodiments can be implemented is illustrated. In particular, as shown, a user might have a number of different devices that are able to communicate using at least one wireless communication protocol. For example, as shown, the user might have a user computing device 102, such as a smartwatch 202 or fitness tracker, which the user would like to be able to communicate with another device, such as smartphone 204, a tablet computer 206, one or more external data sources 154 (e.g., a thermometer for measuring core body temperature, a scale for measuring weight, a health records system, etc.), and/or the like. The ability to communicate with multiple devices can enable a user to view information from the smartwatch 302, e.g., data captured using a sensor on the smartwatch 202, and any other linked source (e.g., external data source 154) using an application installed on either the smartphone 204 or the tablet computer 206. The user may also want the smartwatch 302 to be able to communicate with the server computing system 150 of the service provider, or other such entity, which is able to obtain and process data from the smartwatch and/or any other suitable device (e.g., the external data source(s) 154) to provide functionality that may not otherwise be available on the smartwatch or the applications installed on the individual devices. In addition, as shown, the smartwatch 202 may be able to communicate with the server computing system 150 of the service provider through the at least one network 152, such as the Internet or a cellular network, or may communicate over a wireless connection such as Bluetooth^{®} to one of the individual devices, which can then communicate over the at least one network.

There may be a number of other types of, or reasons for, communications in various embodiments. For instance, a user may want to allow the computing system 100 to access health data from an external health records system (e.g., for a health provider). For instance, the health data can include pre-existing health data of the user in the form of electronic health records that can include biomarker data, age, pre-existing health conditions, etc. Biomarker data could have been previously collected in an invasive or non-invasive manner and can include biomarkers from blood testing, and this data can be related to a complete blood count, a comprehensive metabolic panel, an insulin level, a blood glucose level, a total cholesterol level, an HDL cholesterol level, an LDL cholesterol level, a triglyceride level, an HbAlc level, a high-sensitivity C-reactive protein level, a gamma-glutamyl transferase level, a testosterone level, a blood urea nitrogen level, a creatinine level, an Estimated Glomerular Filtration Rate (eGFR), a sodium level, a potassium level, a chloride level, a carbon dioxide level, a calcium level, a total protein level, an albumin level, a globulin level, an albumin/globulin ratio, a total bilirubin level, an alkaline phosphatase (ALP) level, an aspartate aminotransferase (AST) level, an alanine aminotransferase (ALT) level, or a combination thereof.

In addition to being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, such that no manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth^{®}, and in particular Bluetooth Low Energy (BLE) which has very low power consumption.

In further embodiments, the environment 200 illustrated in FIG. 8 enables data to be captured, processed, and displayed in a number of different ways. For example, data may be captured using sensors on the smartwatch 202, but due to limited resources on the smartwatch 202, the data may be transferred to the smartphone 204 or the server computing system 150 of the service provider (or a cloud resource) for processing, and results of that processing may then be presented back to that user on the smartwatch 202, the smartphone 204, and/or another such device associated with that user, such as the tablet computer 206. In at least some embodiments, a user may also be able to provide input such as health data from an external data source using an interface on any of these devices, which can then be considered when making that determination.

As mentioned above, data generated by the IPG sensor(s) 116 and optionally by the PPG sensor(s) 126 of the user computing device 102 positioned on the housing 104 of the user computing device may be simultaneously monitored to detect blood pressure or blood pressure related parameters. Thus, in such embodiments, consuming both fingertip PPG and fingertip IPG data is configured to increase accuracy of the blood pressure (or its proxy) estimation. In general, the IPG sensor(s) 116, which measures changes in impedance representative of pulsatile changes in blood volume, are less affected by the effects of vasoconstriction and vasodilation in comparison to the PPG sensor(s) 126. Furthermore, PPG data is used in features like heart rate estimation, passive atrial fibrillation detection, and detecting loss of pulse. However, there can be instances when PPG data has a degraded signal, which leads to false detections of heart rate, atrial fibrillation, loss of pulse, etc. Since IPG data is usable to detect pulse, IPG data is usable as a complementary signal to the PPG data to reduce false detections. IPG signals can also be used as a reference signal for PPG to compensate for motion artifacts or applied pressure on the sensors. Accordingly, in certain instances, the combination of IPG data and PPG data can be used to provide a more accurate blood pressure estimation when measured, e.g., via the fingertip.

Once the blood pressure is detected from at least the IPG sensor(s) 116 described herein, it may also be useful to notify the user of the user computing device 102 of this information, such as via a notification on the display 106 and/or through another output device of the user computing device 102 or another computing device (e.g., tablet computer, mobile phone, laptop, personal computer, etc.) so the user can be educated and/or make lifestyle, diet, and/or other changes. In some instances, a user may interact with a conversational interface (e.g., chatbot), which may have access to an artificial intelligence (AI) model (e.g., a machine learned, large language model, and/or the like), via one or more user interfaces (e.g., of the user computing device 102, a laptop, a tablet computer, and/or the like) for a user to ask questions regarding blood pressure metrics and receive feedback from the user interface. In instances where an AI model is accessible, the feedback may be more personalized based on the user's personal metrics depending on user permission settings.

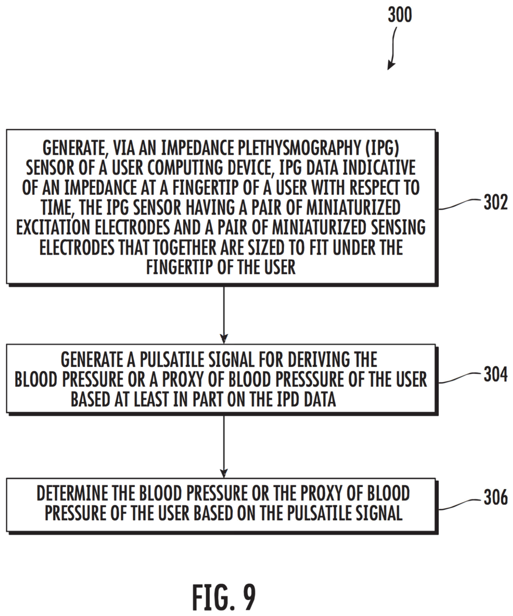

Referring now to FIG. 9, a flow diagram of an embodiment of a method 300 for monitoring blood pressure of a user according to the present disclosure is provided. In general, the method 300 is described herein with reference to the user computing device 102 described in FIGS. 1-8. However, it should be appreciated that the disclosed method 300 may be implemented with any other suitable wearable computing device having any other suitable configurations. In addition, although FIG. 9 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

As shown at (302), the method 300 may include generating, via an impedance plethysmography (IPG) sensor of a user computing device, IPG data indicative of an impedance at a fingertip of a user with respect to time. As mentioned herein, the IPG sensor may include a pair of miniaturized excitation electrodes and a pair of miniaturized sensing electrodes that together are sized to fit under the fingertip of the user. Thus, in an embodiment, generating the IPG data can be completed using a variety of methods, such as brute force methods and/or gating methods. As such, in an embodiment, the IPG sensor(s) described herein can be operated all the time. In another embodiment, the IPG sensor(s) described herein can be operated based on activity levels of other elements in the user computing device (such as from a motion sensor, display activity (e.g., a user is passively engaged with the device such as watching a youtube video and may interact with the sensor at some point), a camera (e.g. passive detection of face-based health screening), communication (e.g. a text message arrived so a user is likely to touch the button soon), and so on). In still further embodiments, the PPG sensor(s) described herein may gate the IPG sensor, e.g., if the PPG sensor(s) detects a few pulses, said sensor is configured to immediately turn on the IPG sensor(s) to get more pulsatile information and/or vice versa. Furthermore, as shown at (304), the method 300 may include generating, via a processor, a pulsatile signal for deriving the blood pressure or a proxy of blood pressure of the user based at least in part on the IPG data. Moreover, as shown at (306), the method may include determining, via the processor, the blood pressure or the proxy of blood pressure of the user based on the pulsatile signal.

### Additional Disclosure

The technology discussed herein makes reference to servers, databases, software applications, and other computer-based systems, as well as actions taken and information sent to and from such systems. The inherent flexibility of computer-based systems allows for a wide variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

In addition, although the figures and description depict and describe steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

Further to the descriptions above, a user may be provided with controls allowing the user to make an election as to both if and when systems, programs, or features described herein may enable collection of user information (e.g., information about a user's health data, activities, social network, social actions, profession, a user's preferences, or a user's current location, etc.), and if the user is sent content or communications from a server. In addition, certain data may be treated in one or more ways before it is stored or used, so that personally identifiable information is removed. For example, a user's identity may be treated so that no personally identifiable information can be determined for the user, or a user's geographic location may be generalized where location information is obtained (such as to a city, ZIP code, or state level), so that a particular location of a user cannot be determined. Thus, the user may have control over what information is collected about the user, how that information is used, and what information is provided to the user.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of an embodiment can be used with another embodiment to yield a still further embodiment. Thus, it is intended that the present disclosure covers such alterations, variations, and equivalents.

## Claims

1. A user computing device, comprising:
a housing;
an impedance plethysmography, IPG, sensor positioned on the housing, the IPG sensor having a pair of miniaturized excitation electrodes and a pair of miniaturized sensing electrodes that together are sized to fit under a fingertip of a user, the IPG sensor being configured to generate IPG data indicative of an impedance at the fingertip of the user with respect to time; and
a processor configured to:
receive the IPG data;
generate a pulsatile signal for deriving a blood pressure or a proxy of blood pressure of the user based at least in part on the IPG data; and
determine the blood pressure or the proxy of blood pressure of the user based on the pulsatile signal.

2. The user computing device of claim 1, further comprising a user interface.

3. The user computing device of claim 1 or 2, wherein the pair of miniaturized excitation electrodes are arranged exterior of the pair of miniaturized sensing electrodes, and wherein the pair of miniaturized excitation electrodes inject alternating current into the fingertip and the pair of miniaturized sensing electrodes measure a resulting voltage.

4. The user computing device of claim 3, wherein the pair of miniaturized excitation electrodes and the pair of miniaturized sensing electrodes are arranged in a linear pattern.

5. The user computing device of any one of the preceding claims, wherein the pair of miniaturized excitation electrodes and the pair of miniaturized sensing electrodes are constructed of a conductive material, and wherein the IPG sensor further comprises an insulating substrate material arranged between the pair of miniaturized excitation electrodes and the pair of miniaturized sensing electrodes and the user computing device, wherein the insulating substrate material is configured to mitigate electrical shorting between the pair of miniaturized excitation electrodes, the pair of miniaturized sensing electrodes, the housing, and one or more internal electronics of the user computing device.

6. The user computing device of claim 5, wherein the pair of miniaturized excitation electrodes and the pair of miniaturized sensing electrodes are raised with respect to the insulating substrate material by a certain height, wherein, optionally, the certain height ranges from about 10 microns to about 100 microns.

7. The user computing device of claim 5 or 6, further comprising electrical circuity electrically coupled with the pair of miniaturized excitation electrodes and the pair of miniaturized sensing electrodes that passes through the insulating substrate material to internal electrodes on a non-fingertip side of the IPG sensor.

8. The user computing device of any one of the preceding claims, wherein the electrical circuitry is further electrically coupled with a bioimpedance module for performing bioimpedance calculations.

9. The user computing device of any one of the preceding claims, further comprising an optical module comprising a photoplethysmography (PPG) sensor positioned on the housing and proximate the IPG sensor, the PPG sensor including an emitter configured to emit light and a detector configured to detect the light emitted from the emitter, the PPG sensor being configured to generate PPG data indicative of an amount of light detected by the detector.

10. The user computing device of claim 9, wherein the PPG sensor is positioned centrally within the IPG sensor between the pair of miniaturized excitation electrodes and the pair of miniaturized sensing electrodes or the PPG sensor is positioned below at least a portion of the IPG sensor.

11. The user computing device of claim 10, wherein at least one of the miniaturized excitation electrodes and the miniaturized sensing electrodes are constructed of a transparent conducting film.

12. The user computing device of claim 11, further comprising electrical circuity electrically coupling the miniaturized excitation electrodes and the miniaturized sensing electrodes constructed of the transparent conducting film to internal electrodes at an underside edge of the miniaturized excitation electrodes and the miniaturized sensing electrodes.

13. The user computing device of any one of the preceding claims, wherein the housing has an upper side and a lower side and a plurality of side walls, the IPG sensor positioned on at least one of the upper side, one of the plurality of side walls, or the lower side.

14. The user computing device of any one of the preceding claims, wherein the user computing device comprises at least one of a smartphone, a smartwatch, a tablet computer, a laptop, earbuds, a headset, headphones, or gaming controllers.

15. A method for monitoring blood pressure of a user, the method comprising:
generating, via an impedance plethysmography, IPG, sensor of a user computing device, IPG data indicative of an impedance at a fingertip of a user with respect to time, the IPG sensor having a pair of miniaturized excitation electrodes and a pair of miniaturized sensing electrodes that together are sized to fit under the fingertip of the user;
generating, via a processor, a pulsatile signal for deriving the blood pressure of the user based at least in part on the IPG data; and
determining, via the processor, the blood pressure of the user based on the pulsatile signal.
